# EUROPEAN PATENT APPLICATION

(11) **EP 4 792 840 A2**
(43) Date of publication of application: **19.08.2026**
(21) Application number: 26170837.4
(22) Date of filing: 28.04.2023
(51) Int. Cl.: A61L 26/00

(54) **COMPOSITION FOR SEALING AROUND A STOMA**

(30) Priority: 28.04.2022 DK PA202200406
(62) Document number(s) of the earlier application(s) in accordance with Art. 76 EPC: 23723133.7 / 4 514 410
(71) Applicant: Coloplast A/S, 3050 Humlebaek (DK)
(72) Inventor: Vange, Jakob, 3050 Humlebaek (DK); Stroebech, Esben, 3050 Humlebaek (DK); Larsen, Esben Kjaer Unmack, 3050 Humlebaek (DK)

(57) **Abstract**

A cellulose composition for sealing around a stoma, an ostomy device or an accessory for an ostomy device comprising the composition, and a method for sealing around a stoma. The composition comprises a continuous phase and a discontinuous phase, with the continuous phase comprising a cellulose derivate material and a plasticizer and the discontinuous phase comprising an absorbent material. The cellulose derivate material is soluble in the plasticizer, and the composition comprises 15-40 % (w/w) cellulose derivative material, 10-30 % (w/w) plasticizer; and 40-70 % (w/w) absorbent material.

## Description

The invention relates to compositions for sealing around a stoma as well as ostomy devices comprising such compositions and methods of using such compositions.

### Background

In connection with treatment for diseases of the gastro-intestinal tract, parts or all of the colon, the ileum or the urethra has to be surgically removed or temporarily bypassed. The patient is left with an abdominal stoma, either temporary or permanent, and the effluents or waste products of the body are discharged through the stoma and collected in a collection bag. The bag is usually adhered to the skin by means of an adhesive wafer or plate having an inlet opening for accommodating the stoma. A separate ring-shaped adhesive and/or an adhesive paste may be used as an accessory for filling an area between the stoma and the adhesive wafer.

### Detailed description of the Invention

In the following, whenever referring to proximal side of a device or part of a device, the referral is to the skin-facing side, when the ostomy appliance is worn by a user. Likewise, whenever referring to the distal side of a device or part of a device, the referral is to the side facing away from the skin, when the ostomy appliance is worn by a user. In other words, the proximal side is the side closest to the user, when the appliance is fitted on a user and the distal side is the opposite side - the side furthest away from the user in use.

Embodiments provide a cellulose composition for sealing around a stoma, the composition comprising: 20-40 % (w/w) of cellulose derivative material; 10-30 % (w/w) of plasticizer; and 40-70 % (w/w) of absorbent material. As shown in the Examples, a composition of this type achieves the desirable swelling characteristics to properly and quickly be able to seal around a stoma.

The composition is a cellulose composition, which means that one or more cellulose derivative materials make up a significant part of the composition.

The composition is for sealing around a stoma, which means that it is suitable for being applied to or in proximity of the skin of a person around a stoma and create a seal between the stoma and the composition. The object of this sealing effect is to prevent leakage of output from the stoma onto the skin of the person.

The cellulose derivative material of the composition can be said to form a matrix in which the other components are incorporated. In other words, the composition comprises a matrix, sometimes also referred to as a continuous phase, and some discrete components within the matrix, which components can be referred to as a discontinuous phase or a discrete phase. Embodiments provide a cellulose composition comprising a continuous phase and a discontinuous phase, wherein the continuous phase comprises a cellulose derivative material and a plasticizer and wherein the discontinuous phase comprises an absorbent material. In embodiments, the continuous phase is hydrophilic. In embodiments, the cellulose derivate material is soluble in the plasticizer. For instance, the cellulose derivative hydroxypropyl cellulose (HPC), which can be a component of the continuous phase, is soluble in polyethylene glycol with an average molecular weight of 200 Dalton (PEG200), which is a plasticizer. In embodiments, the absorbent material is not soluble in the plasticizer. For instance, carboxymethyl cellulose (CMC), which is an absorbent material and a possible component of the discontinuous phase, is not soluble in PEG200.

In the present context, solubility refers to the ability of one component of the composition to be dissolved by another component of the composition. If for instance the cellulose derivate material is soluble in the plasticizer, this means that the plasticizer in the composition is capable of dissolving the cellulose derivate material in the composition. Conversely, if something is not soluble, this means that one component of the composition cannot be dissolved by another component of the composition. For instance, if the absorbent material is not soluble in the plasticizer, this means that the plasticizer in the composition cannot dissolve the absorbent material. The inventors are aware that solubility is a relative term: Only a certain amount of material can be dissolved in a given amount of solvent. In the present context, solubility is to be seen in the context of the composition itself. That the cellulose derivate material of the composition is soluble in the plasticizer does not mean that any amount of cellulose derivate material can be dissolved in any amount of plasticizer. It only means that the cellulose derivate material *in the composition* can be dissolved by the plasticizer *in the composition*.

Embodiments provide a cellulose composition for sealing around a stoma, the composition comprising a continuous phase and a discontinuous phase, wherein the continuous phase comprises a cellulose derivate material and a plasticizer and wherein the discontinuous phase comprises an absorbent material, and wherein the cellulose derivate material is soluble in the plasticizer, and wherein the composition comprises 20-40 % (w/w) of the cellulose derivative material, 10-30 % (w/w) of the plasticizer, and 40-70 % (w/w) of the absorbent material.

The composition is water swellable, which means that it swells in the presence of water. Water swelling is an increase in volume of the composition as a consequence of water being taken up by the composition. The water swelling can be caused by the cellulose derivative matrix itself taking up water and/or by the absorbent material absorbing water. Swelling requires an increase in volume of the composition, which is in contrast to, e.g., a sponge or other porous material taking up water by incorporating it into existing cavities or pores, without this water uptake leading to an increased volume of the material. The swelling of the composition, and the resulting increase in volume, is what leads to the sealing effect. The composition expands in volume and is able to fill a gap and provide a seal around, e.g., a stoma. The swelling is a result of the absorbent material, and possibly also the cellulose derivative matrix, absorbing liquid. The inventors have found that the disclosed mix of cellulose derivative matrix, plasticizer and absorbent material provides the right balance between quick swelling and sufficient cohesion.

In embodiments, the composition is mouldable, meaning that it can be permanently deformed by applying pressure. The mouldability of the composition can be adjusted for instance by increasing the amount of continuous phase, which will make it softer and more mouldable. On the other hand, increasing the amount of discontinuous phase will generally make the composition harder and less mouldable.

The present inventors have found that it is difficult to obtain a good and effective sealing around a stoma. On the one hand, the seal must be quite close fitting to prevent leakage. On the other hand, the stoma is typically very sensitive and does not tolerate high pressure or materials rubbing against it. The stoma can easily become irritated, which may cause additional secretions from the stoma. Furthermore, it is important to establish the seal around the stoma as quickly as possible after applying the composition to the skin. If the seal is established too slowly, there will be time for output and secretions from the stoma to leak onto the skin of the person before the seal is established. This means that the skin and anything attached to the skin, such as an ostomy adhesive wafer, will be compromised already from the very beginning by output or secretions on the skin. And this again means that the risk of skin damage and possible failure of the attachment to the skin of other devices is increased dramatically.

When using an ostomy product, such as an ostomy bag mounted on an adhesive baseplate, there will often be a gap between the product and the stoma. In this gap, skin is potentially exposed to output from the stoma. Because output contains surface active bile salts and proteolytic enzymes, this can damage the skin. The adhesive part of the stoma product typically contains various absorbing and gelling compounds and when exposed to the moisture in the output will start to swell and over time reduce the gap. This process is however very slow and may not close the gap enough and fast enough to prevent skin damage.

Realising all this, the inventors have devised the present composition to provide a quick sealing effect around the stoma while still being gentle enough to not damage or irritate the stoma.

The composition comprises a matrix, which is the backbone of the composition. The matrix is typically cohesive enough to maintain sufficient structural integrity of the composition and prevent it from falling apart or dissolving when exposed to liquid for shorter periods of time, such as 2-12 hours. The matrix comprises a cellulose derivative material.

The composition further comprises a plasticizer, which has the effect of plasticizing or softening the composition and giving it the desired rheological properties.

The composition further comprises an absorbent material. The absorbent material can absorb liquid, such as water.

In embodiments, the composition is a non-adhesive composition. By non-adhesive is meant that the composition cannot on its own remain properly adhered to a surface, such as the skin of a person. In embodiments, non-adhesive means that the composition exhibits a peel force below 1 N/25 mm when measured by the peel force test method described herein. Such a peel force below 1 N/25 mm means that the composition can be very easily removed from, e.g., the skin of a person without causing any damage. In order to make sure a non-adhesive composition remains in place it can, e.g., be incorporated into a multi-component product which can itself remain adhered. Or the composition may be secured to the skin by means of other adhesive components, such as the adhesive on a base plate of an ostomy device.

In embodiments, the cellulose derivative material is selected from the group consisting of hydroxypropyl cellulose, methyl hydroxypropyl cellulose, ethyl cellulose and methyl hydroxyethyl cellulose.

In embodiments, the plasticizer is selected from the group consisting of di-, tri-, and tetraethylene glycol, polyethylene glycol, dipropylene glycol, tripropylene glycol, polypropylene glycol, glycerol, glyceryl glucoside, diglycerin, triglycerin, polyglycerin, ethoxylated glycerol, ethoxylated methylglycosid, monoacetin, diacetin, triacetin, aliphatic diols, triols, and polyols, xylitol, erythritol, sorbitol, mannitol, inositol, dibutyltartrate, diethyltartrate, choline, urea, hydroxyethyl urea, PPG-10 methyl glucose ether, aliphatic esters of di- and tri-carboxylic acids and aliphatic esters of fatty acids. In embodiments, the plasticizer is selected from liquid rosin derivatives, aromatic olefin oligomers, vegetable and animal oils and derivatives, polar oils, such as esters, ethers and glycols, poly propylene oxides, such as alpha-butoxy-polyoxypropylene, mineral oil, citrate oil, paraffin oil, phatalic acid esters, adepic acid esters (such as DOA), and liquid or solid resins.

In embodiments, the absorbent material is selected from the group consisting of hydrocolloids, starch, water soluble salts, mono, di- and oligosaccharides, sugar alcohols, polypeptides, organic acids, inorganic acids, amino acids, amines, urea, super absorbent particles, such as polyacrylic acid, glycols, such as polyethylene glycol, fumed silica and bentone. In embodiments, the absorbent material is selected from the group consisting of carboxymethyl cellulose, methylcellulose, hydroxypropyl methylcellulose, potato starch, corn starch, modified starch, hydroxyethyl starch, hydroxypropyl di-starch phosphate, pectin, guar gum, locust bean gum, tara gum, agar, alginates, sodium alginate, calcium alginate, carrageenan, gellan gum, xanthan gum, gelatine, cellulose derivatives, salts of carboxymethyl cellulose, sodium carboxymethyl cellulose, methyl cellulose, hydroxypropyl cellulose, hydroxyethyl cellulose, sodium starch glycolate, and polyvinylalcohol. In embodiments, the absorbent material is hydroxyethylcellulose with a weight average molecular weight in the range 500,000-2,000,000 g/mol, such as 750,000-1,500,000 g/mol, such as 800,000-1,200,000 g/mol, such as 1,000,000 g/mol. In embodiments, the absorbent material is Natrosol 250HX hydroethylcellulose from Ashland.

In embodiments, the cellulose derivate material and the absorbent material are different materials.

In embodiments, the composition comprises 15-40, 15-25, 15-20, 20-30, 30-40, 20-25, 25-30, 30-35, or 35-40 % (w/w) of cellulose derivative material.

In embodiments, the composition comprises 15-30, 20-30, 25-30, 15-25, or 15-20 % (w/w) of plasticizer.

In embodiments, the composition comprises 40-60, 40-50, 50-60, 60-70, 50-55, 50-65, 55-60, 60-65, or 65-70 % (w/w) of absorbent material.

In an embodiment, the composition comprises or consists essentially of 15-25 % (w/w) of cellulose derivative material, 15-25 % of plasticizer, and 50-65 % absorbent material.

In embodiments, the composition further comprises an antioxidant, such as butylated hydroxytoluene. Antioxidants can be particularly helpful when using ethers in the composition. In embodiments, the composition comprises an antioxidant in an amount of less than 5, such as less than 2, such as less than 1, such as less than 0.5 % (w/w).

In embodiments, the composition further comprises one or more filler materials, such as calcium carbonate, magnesium oxide, magnesium hydroxide, aluminium hydroxide, fumed silica, and lignin. The filler material may comprise a non-reinforcing filler such as talc, calcium carbonate, wood powder, or precipitated or fumed silica. More specifically, examples of such fillers include: calcium carbonate (such as dry ground grades of calcium carbonate, wet ground grades of calcium carbonate, beneficiated grades of calcium carbonate, precipitated grades of calcium carbonate, surface treated grades of calcium carbonate); kaolin and other clay-based minerals; talc (such as dry ground talc, calcined talc); quartz and silica, including natural silicas (such as crystalline silica, fused silica, microcrystalline silica, microcrystalline novaculite, diatomaceous silica, perlite) or synthetic silicas (such as fumed silica***,*** precipitated silicas); mica (including ground grades of mica, white grades of mica, surface-modified grades of mica, metal-coated mica grades); metal oxides and other compounds (such as titanium dioxide, alumina trihydrate, wollastonite, barium sulphate, antimony oxide, magnesium oxide, magnesium hydroxide, calcium sulphate, anhydrous calcium sulphate, dihydrate calcium sulphate, feldspar and nepheline syenite); microspheres, solid microspheres, hollow microspheres (such as coated hollow microsphere fillers, metalite aluminium microspheres, polymer-encapsulated gas microspheres); synthetic silicates (such as aluminium silicate, mullite, sillimanite, cyanite, andalusite, synthetic alkali metal aluminosilicates, calcium silicate, magnesium silicate, zirconium silicate). Optional fillers include, for example, calcium carbonate, magnesium oxide, fumed hydrophobic silica, or lignin. Suitable hydrophobic fumed silicas include, for example AEROSIL^{®} R812 (BET surface area g/cm² 230-290), AEROSIL^{®} R974 (BET surface area g/cm² 150-190) or AEROSIL^{®} 200 (BET surface area g/cm² 200 ± 25).

In embodiments, the composition is in the shape of a ring. A ring shape is especially useful for providing sealing all the way around a stoma. The composition may alternatively be in the form of a half ring, in which case the user may apply one or two half rings according to need and preference.

In embodiments, the ring has an outer diameter of 40-100 mm, such as 50-80 mm, such as 50-60 mm, and a central hole with a diameter of 5-30 mm, such as 10-20 mm.

In embodiments, the ring has a thickness of 0.2-3 mm, such as 0.3-2 mm, such as 0.5-1.5 mm.

In embodiments, the composition has a swelling of at least 0.75 mm, 1 mm, 1.25 mm or 1.5 mm after 30 minutes when measured as according to the swelling speed test method described in the specification. This swelling speed is preferable in terms of quite quickly sealing around the stoma and minimizing the risk of output leaking onto the skin of the user. In embodiments, the swelling can be at least 2 mm after 1 hour.

In embodiments, the composition comprises 15-25 % (w/w) of cellulose derivative material, 15-25 % (w/w) of plasticizer and 45-70 % (w/w) of absorbent material.

In embodiments, the composition consists essentially of 15-25 % (w/w) of cellulose derivative material, 15-25 % (w/w) of plasticizer and 45-70 % (w/w) of absorbent material.

In embodiments, the composition consists of 15-25 % (w/w) of cellulose derivative material, 15-25 % (w/w) of plasticizer and 45-70 % (w/w) of absorbent material.

In embodiments, the cellulose derivate material is hydroxypropyl cellulose. In embodiments, the absorbent material comprises carboxymethyl cellulose and polyacrylic acid. In embodiments, the plasticizer comprises polyethylene glycol with a molecular weight of 200 Dalton.

Provided is an ostomy device comprising an adhesive for attaching the device to the skin of a user, a release liner on the proximal side of the adhesive, a backing layer on the distal side of the adhesive, a hole for accommodating a stoma and a collecting bag attached to the backing layer, wherein the ostomy device further comprises a composition as described herein. In embodiments, the composition is disposed on the distal side of the backing layer. In embodiments, the composition is disposed between the adhesive and the backing layer.

Provided is an accessory for an ostomy device comprising an adhesive for attaching the accessory to the skin around a stoma of a user, a hole for accommodating the stoma and a composition as described herein disposed on a distal side of the adhesive. In embodiments, the accessory further comprises a release liner on a proximal side of the adhesive. In embodiments, the accessory further comprises a backing layer on the distal side of the composition.

Provided is a method for sealing around a stoma, the method comprising the steps of providing a composition as described herein; applying the composition around the stoma; and allowing the composition to swell, thereby sealing around the stoma.

### Items

1. A cellulose composition for sealing around a stoma, the composition comprising:
   - 20-40 % (w/w) of cellulose derivative material;
   - 10-30 % (w/w) of plasticizer; and
   - 40-70 % (w/w) of absorbent material.
2. The composition of item 1, wherein the composition is water-swellable.
3. The composition of item 1 or 2, wherein the composition is non-adhesive.
4. The composition of any one of the preceding items, wherein the cellulose derivative material is selected from the group consisting of hydroxypropyl cellulose, methyl hydroxypropyl cellulose, ethyl cellulose and methyl hydroxyethyl cellulose.
5. The composition of any one of the preceding items, wherein the absorbent material is selected from the group consisting of hydrocolloids, starch, water soluble salts, mono, di- and oligosaccharides, sugar alcohols, polypeptides, organic acids, inorganic acids, amino acids, amines, urea, super absorbent particles, glycols, fumed silica, and bentone.
6. The composition of any one of the preceding items, wherein the composition is in the shape of a ring.
7. The composition of any one of the preceding items, wherein the composition has a swelling of at least 1.5 mm after 30 minutes when measured as according to the swelling speed test method described in the specification.
8. The composition of any one of the preceding items, wherein the composition comprises 15-25 % (w/w) of cellulose derivative material, 15-25 % (w/w) of plasticizer and 45-70 % (w/w) of absorbent material.
9. The composition of any one of the preceding items, wherein the composition consists essentially of 15-25 % (w/w) of cellulose derivative material, 15-25 % (w/w) of plasticizer and 45-70 % (w/w) of absorbent material.
10. An ostomy device comprising an adhesive for attaching the device to the skin of a user, a release liner on the proximal side of the adhesive, a backing layer on the distal side of the adhesive, a hole for accommodating a stoma and a collecting bag attached to the backing layer, wherein the ostomy device further comprises a composition according to any one of the preceding claims.
11. The ostomy device of item 10, wherein the composition is disposed on the distal side of the backing layer or between the adhesive and the backing layer.
12. An accessory for an ostomy device comprising an adhesive for attaching the accessory to the skin around a stoma of a user, a hole for accommodating the stoma and a composition according to any one of items 1-9 disposed on a distal side of the adhesive.
13. The accessory device of item 12, wherein the accessory further comprises a release liner on a proximal side of the adhesive.
14. The accessory device of item 12 or 13, wherein the accessory further comprises a backing layer on the distal side of the composition.
15. A method for sealing around a stoma, the method comprising the steps of
   - providing a composition according to any one of items 1-9;
   - applying the composition around the stoma; and
   allowing the composition to swell, thereby sealing around the stoma.

### Test Methods

### Peel force test method

To perform peel tests, a sample was provided in a rectangular shape sized 25 mm x 100 mm. The sample was firmly pressed on to a thoroughly cleaned Teflon plate. A 25x300 mm² piece of auxiliary tape was then placed on the top of the sample and the whole sample pressure rolled to assure firm adhesion between the tape and the sample to be tested. After conditioning for 30 minutes at 23 centigrade, the sample was mounted in a tensile testing machine and a 90 degrees peel test was carried out at a speed of 304 mm/min. The result is given in N/25 mm.

### Swelling speed test method

Swelling speed was tested by hot pressing the composition to be tested at 90 °C for 30 sec to a thickness of 1 mm between sheets of release liner (siliconized paper). From this, a ring (outer diameter 57 mm, hole diameter 27 mm) was cut.

A hard plastic (polyoxymethylene, "POM") disc (diameter 17 mm, 3 mm thick) was attached with double sided adhesive tape in the center of a glass petri dish. The ring of swellable composition was mounted in the glass petri dish centred around the POM disc to give a 5 mm gap between the POM and the ring of swellable composition.

When isotonic saline is added to sample to initiate swelling, the process is an inherently low contrast event. To increase contrast, crossed polarizing filters were used: A sheet of polarizing film was attached to a light table. The sample was placed on top of this film. A camera (Canon EOS 1200D) was mounted to observe sample from above. On the lens of the camera was mounted another polarizing filter. This filter was then adjusted to let the least amount of light through (this happens when the 2 filters are at 90° orientation to each other). First picture of sample was taken, saline was added to cover the ring and subsequent pictures were taken at pre-determined time points, such as 1-24 hours. The gap between the ring and the POM disc at positions 12-3-6-9 o'clock was now measured using ImageJ, both at start (t0) and at later time points. The average change in gap size was calculated and reported in mm at the different time points.

### Erosion resistance test method

To test erosion resistance, the composition was hot pressed to 0.5 mm between 2 sheets of release liner (siliconized paper). Square samples (25x25 mm) were made and a triangle-shaped notch of the material (approx. 10x10x10 mm) was cut out of the top part of the square with the tip of the triangle pointing towards the center of the square. The notched square was mounted on a glass plate. This glass plate was then mounted in an upright position (~10° from vertical) in front of a light table (also in the upright position) with a polarizing filter (as in the swelling speed test method, above). The sample was observed with a camera with a polarizing filter. The filter was adjusted to maximum darkness. A reservoir with saline was placed under the sample, and from this reservoir, saline was pumped to the notches in the samples at 4 mL/min. Time lapse photography was started, and the time it took for the flowing liquid to carve a clear channel through the material was used as a measure of erosion resistance.

### Examples

The following compositions were produced. CMC is carboxymethyl cellulose sodium salt and GR-221 is sodium polyacrylic acid superabsorber powder, both being absorbent materials. PEG200 is polyethylene glycol with an average molecular weight of 200 Dalton, which is a plasticizer. HPC is hydroxypropyl cellulose. BHT is butylated hydroxytoluene, which is an antioxidant.

**Table 1**

| Recipe | CMC (%wt) | GR-221 (%wt) | PEG200 (%wt) | HPC (%wt) | BHT (%wt) | HPC grade | HPC molecular weight (kDa) |
|---|---|---|---|---|---|---|---|
| A | 32.5 | 32.5 | 17.4 | 17.4 | 0.2 | Klucel LF Pharm (Ashland) | 95 |
| B | 32.5 | 32.5 | 17.4 | 17.4 | 0.2 | Klucel JF Pharm (Ashland) | 140 |
| C | 32.5 | 32.5 | 17.4 | 17.4 | 0.2 | Klucel GF Pharm (Ashland) | 370 |
| D | 32.5 | 32.5 | 17.4 | 17.4 | 0.2 | Klucel MF Pharm (Ashland) | 850 |
| E | 32.5 | 32.5 | 17.5 | 17.5 | 0 | Klucel LF Pharm (Ashland) | 95 |
| F | 27.5 | 27.5 | 22.5 | 22.5 | 0 | Klucel LF Pharm (Ashland) | 95 |
| G | 25 | 25 | 25 | 25 | 0 | Klucel LF Pharm (Ashland) | 95 |

Recipes A, B, C and D were made to test HPC grades with different molecular weights. These recipes were all produced as set out in Example 3 below with the BHT added to the matrix mix produced according to Example 1, except with varying grades of the HPC component.

Recipe E is the exact recipe resulting from Example 3 below.

Recipes F and G were produced in the same way as described in Example 3 but with varying content of the different components.

### Example 1: Hydroxypropyl cellulose-based matrix

34.5 g PEG200 was added to a glass beaker. Then 34.5 g hydroxypropyl cellulose (Klucel LF PHARM, Ashland) was added with stirring. The mixture was left for 1 hour at room temperature to set, then hot-mixed (Brabender) at 80 °C for 40 minutes at 50 rpm. This resulted in a soft, sticky paste at room temperature.

### Example 2: Ethyl cellulose-based matrix

34.5 g tributyl citrate was added to a glass beaker. Then 34.5 g ethyl cellulose (Aqualon EC N4, Ashland) was added with stirring. The mixture was left to set for 1 hour at room temperature and then hot-mixed (Brabender) at 80 °C for 40 minutes at 50 rpm. This resulted in a soft, tacky paste at room temperature

### Example 3: Swellable composition with hydroxypropyl cellulose-based matrix binder

21 g matrix paste of Example 1, 19.5 g carboxy methylcellulose (Akucell AF2881, Nouryon) and 19.5 g sodium polyacrylic acid (GR-221 superabsorber powder, Chase Corporation) were added to a Speedmixer cup and mixed at 2000 rpm (3x1min) (Speedmixer DAC150.1 FVZ (Hauschild)). The result was a soft paste when hot, which solidified to a firm paste when cooled to room temperature.

### Example 4: Swellable composition with ethyl cellulose-based matrix

21 g matrix of Example 2, 19.5 g carboxy methylcellulose (Akucell AF2881, Nouryon) and 19.5 g sodium polyacrylic acid (GR-221 superabsorber powder, Chase Corporation) were added to a Speedmixer cup and mixed at 2000 rpm (3x1 min) (Speedmixer DAC150.1 FVZ (Hauschild)). This resulted in a soft paste when hot, which solidified to a firm paste when cooled to room temperature.

### Example 5: Comparing swelling speed of commercial ostomy sealing ring and swellable composition "Recipe B"

The swelling speed of a commercial ostomy sealing ring (hydrophobic adhesive matrix with hydrocolloids) was measured as described herein above in the "Swelling speed test method" section. This commercial product was a hydrophobic adhesive with hydrophilic filler particles. Swelling speed was measured at 0, 1.5, 4, 8, 12 and 16 hours.

Similarly, the swellable composition according to Recipe B in Table 1 was tested by the "Swelling speed test method" described herein with measurements taken at 15, 30, 60 and 120 minutes. The measurements were taken earlier and at shorter intervals due to an observed much faster swelling of Recipe B.

The results comparing swelling speed of the commercial ostomy seal and Recipe B are shown in Table 2. All reported results are averages of four individual samples. The results in parentheses are calculated values based on a curve fitted to the measured results.

**Table 2**

| **Sample** | **0 min** | **15 min** | **30 min** | **1 h** | **1.5 h** | **2 h** | **4 h** | **8 h** | **12 h** | **16h** |
|---|---|---|---|---|---|---|---|---|---|---|
| Commercial ostomy seal | 0 | (0.3 mm) | (0.4 mm) | (0.6 mm) | 0.8 mm | (0.9 mm) | 1.3 mm | 1.8 mm | 2.2 mm | 2.6 mm |
| Recipe B | 0 | 1.6 mm | 2.0 mm | 2.6 mm | (3.0 mm) | 3.5 mm | - | - | - | - |

The results in Table 2 show that the commercial ostomy seal product swells quite slowly and therefore takes a long time to close the gap between the adhesive and the POM disc. Even after 4 hours, the ostomy seal has only swelled 1.3 mm. Translated to a use situation where the POM disc simulates the stoma and the gap simulates exposed skin, the skin will be exposed to stomal output for a very long time before it is sealed off by the slowly swelling adhesive. This contrasts with the swelling composition of Recipe B, which has swelled 2.6 mm after only 1 hour. And even after only 15 minutes, the composition of Recipe B has swelled 1.6 mm, thereby very quickly working to close the gap between the composition as applied and the POM disc.

### Example 6: Erosion resistance

The erosion resistance of the compositions was tested as described in the erosion resistance test method described herein above. Recipes A-D were tested to better understand the effect of different HPC grades and recipes E-G were tested to examine the effect of different amounts of HPC in the compositions. The results are shown in Table 3 with erosion resistance reported as the time it takes for liquid to carve a channel through the material. A higher number represent a higher erosion resistance.

**Table 3**

| Recipe | HPC (%wt) | HPC grade | HPC molecular weight (kDa) | Erosion resistance (time to failure) |
|---|---|---|---|---|
| A | 17.4 | Klucel LF Pharm (Ashland) | 95 | 4.7 hours |
| B | 17.4 | Klucel JF Pharm (Ashland) | 140 | 6.2 hours |
| C | 17.4 | Klucel GF Pharm (Ashland) | 370 | 10.1 hours |
| D | 17.4 | Klucel MF Pharm (Ashland) | 850 | 15.6 hours |
| E | 17.5 | Klucel LF Pharm (Ashland) | 95 | 4.7 hours |
| F | 22.5 | Klucel LF Pharm (Ashland) | 95 | 3.5 hours |
| G | 25 | Klucel LF Pharm (Ashland) | 95 | 2.9 hours |

The results in Table 3 show that a higher molecular weight of the HPC material leads to an increased erosion resistance. On the other hand, an increased content of HPC (and plasticizer) relative to absorbers leads to a decreased erosion resistance. This information can be used to fine-tune the properties of a composition for commercial use. The used test method does not translate directly into the speed of erosion that would be expected if the composition was worn on the skin of, e.g., an ostomy user. However, all the compositions tested are deemed to have acceptable levels of erosion resistance in terms of being suitable for use as a composition for sealing around a stoma.

### Embodiments

1. A cellulose composition for sealing around a stoma, the composition comprising a continuous phase and a discontinuous phase, wherein the continuous phase comprises a cellulose derivate material and a plasticizer and wherein the discontinuous phase comprises an absorbent material, and wherein the cellulose derivate material is soluble in the plasticizer, and wherein the composition comprises:
   - 15-40 % (w/w) of the cellulose derivative material,
   - 10-30 % (w/w) of the plasticizer, and
   - 40-70 % (w/w) of the absorbent material.
2. The composition of embodiment 1, wherein the absorbent material is not soluble in the plasticizer.
3. The composition of embodiment 1 or 2, wherein the composition is water-swellable.
4. The composition of any one of the preceding embodiments, wherein the composition is non-adhesive.
5. The composition of any one of the preceding embodiments, wherein the cellulose derivative material is selected from the group consisting of hydroxypropyl cellulose, methyl hydroxypropyl cellulose, ethyl cellulose and methyl hydroxyethyl cellulose.
6. The composition of any one of the preceding embodiments, wherein the absorbent material is selected from the group consisting of hydrocolloids, starch, water soluble salts, mono, di- and oligosaccharides, sugar alcohols, polypeptides, organic acids, inorganic acids, amino acids, amines, urea, super absorbent particles, glycols, fumed silica, and bentone.
7. The composition of any one of the preceding embodiments, wherein the composition is in the shape of a ring.
8. The composition of any one of the preceding embodiments, wherein the composition has a swelling of at least 0.75 mm after 30 minutes when measured as according to the swelling speed test method described in the specification.
9. The composition of any one of the preceding embodiments, wherein the composition comprises 15-25 % (w/w) of cellulose derivative material, 15-25 % (w/w) of plasticizer and 45-70 % (w/w) of absorbent material.
10. The composition of any one of the preceding embodiments, wherein the composition consists essentially of 15-25 % (w/w) of cellulose derivative material, 15-25 % (w/w) of plasticizer and 45-70 % (w/w) of absorbent material.
11. An ostomy device comprising an adhesive for attaching the device to the skin of a user, a release liner on the proximal side of the adhesive, a backing layer on the distal side of the adhesive, a hole for accommodating a stoma and a collecting bag attached to the backing layer, wherein the ostomy device further comprises a composition according to any one of the preceding embodiments.
12. The ostomy device of embodiment 11, wherein the composition is disposed on the distal side of the backing layer or between the adhesive and the backing layer.
13. An accessory for an ostomy device comprising an adhesive for attaching the accessory to the skin around a stoma of a user, a hole for accommodating the stoma and a composition according to any one of embodiments 1-10 disposed on a distal side of the adhesive.
14. The accessory device of embodiment 13, wherein the accessory further comprises a release liner on a proximal side of the adhesive.
15. The accessory device of embodiment 13 or 14, wherein the accessory further comprises a backing layer on the distal side of the composition.
16. A method for sealing around a stoma, the method comprising the steps of
   - providing a composition according to any one of embodiments 1-10,
   - applying the composition around the stoma, and
   - allowing the composition to swell, thereby sealing around the stoma.

## Claims

1. A cellulose composition for sealing around a stoma, the composition comprising a continuous phase and a discontinuous phase, wherein the continuous phase comprises a cellulose derivate material and a plasticizer and wherein the discontinuous phase comprises an absorbent material, and wherein the cellulose derivate material is soluble in the plasticizer, and wherein the composition comprises:
- 15-40 % (w/w) of the cellulose derivative material,
- 10-30 % (w/w) of the plasticizer, and
- 40-70 % (w/w) of the absorbent material, wherein
the composition is in the shape of a ring, the ring having an outer diameter of 40-100 mm, such as 50-80 mm, such as 50-60 mm, and a central hole with a diameter of 5-30 mm, such as 10-20 mm.

2. The composition of claim 1, wherein the absorbent material is not soluble in the plasticizer.

3. The composition of claim 1 or 2, wherein the composition is water-swellable.

4. The composition of any one of the preceding claims, wherein the composition is non-adhesive.

5. The composition of any one of the preceding claims, wherein the cellulose derivative material is selected from the group consisting of hydroxypropyl cellulose, methyl hydroxypropyl cellulose, ethyl cellulose and methyl hydroxyethyl cellulose.

6. The composition of any one of the preceding claims, wherein the absorbent material is selected from the group consisting of hydrocolloids, starch, water soluble salts, mono, di- and oligosaccharides, sugar alcohols, polypeptides, organic acids, inorganic acids, amino acids, amines, urea, super absorbent particles, glycols, fumed silica, and bentone.

7. The composition of any one of the preceding claims, wherein the composition has a swelling of at least 2 mm after 1 hour when measured as according to the swelling speed test method described in the specification.

8. The composition of any one of the preceding claims, wherein the ring has a thickness of 0.2-3 mm, such as 0.3-2 mm, such as 0.5-1.5 mm.

9. The composition of any one of the preceding claims, wherein the composition further comprises one or more filler materials.

10. The composition of claim 9, wherein the one or more filler materials comprises a non-reinforcing filler.

11. An ostomy device comprising an adhesive for attaching the device to the skin of a user, a release liner on the proximal side of the adhesive, a backing layer on the distal side of the adhesive, a hole for accommodating a stoma and a collecting bag attached to the backing layer, wherein the ostomy device further comprises a composition according to any one of the preceding claims.

12. The ostomy device of claim 11, wherein the composition is disposed on the distal side of the backing layer or between the adhesive and the backing layer.

13. A method for sealing around a stoma, the method comprising the steps of
- providing a composition according to any one of claims 1-10,
- applying the composition around the stoma, and
- allowing the composition to swell, thereby sealing around the stoma.
